# EUROPÄISCHE PATENTSCHRIFT

(11) **EP 1 121 075 B1**
(45) Veröffentlichungstag und Bekanntmachung des Hinweises auf die Patenterteilung: **09.06.2004**
(21) Anmeldenummer: 98946211.4
(22) Anmeldetag: 15.10.1998
(51) Int. Cl.: A61F 2/44

(54) **TELESKOPIERENDE WIRBELPROTHESE**
TELESCOPIC VERTEBRAL PROSTHESIS
PROTHESE VERTEBRALE TELESCOPIQUE

(43) Veröffentlichungstag der Anmeldung: 08.08.2001
(73) Patentinhaber: SYNTHES AG Chur, 7002 Chur (CH)
(72) Erfinder: LÄNG, Bruno, CH-4557 Horriwil (CH); BENOIT, Alfred, CH-2543 Lengnau (CH)
(74) Vertreter: Lusuardi, Werther Giovanni, Dr.
(86) Internationale Anmeldenummer: PCT/CH1998/000441
(87) Internationale Veröffentlichungsnummer: WO 2000/023013

(56) Entgegenhaltungen:
- EP-A- 0 290 767
- WO-A-96/17564
- DE-A- 2 750 648
- DE-A- 3 023 942
- DE-A- 19 604 246
- DE-A- 19 622 827
- US-A- 5 330 535

## Beschreibung

Die Erfindung bezieht sich auf eine Vorrichtung zum Ersatz von Wirbelkörpern der menschlichen Wirbelsäule gemäss dem Oberbegriff des Patentanspruchs 1.

Wird ein Wirbelkörper krank oder erleidet er einen Defekt, so muss dieser aus der Wirbelsäule entfernt werden. Aus dem Stand der Technik sind einige distanzhaltende Implantate zum Ersatz des fehlenden Wirbelkörpers bekannt. Solche Implantate umfassen üblicherweise gegeneinander verschiebbare Teile, die unter anderem mittels Verzahnungen eine Einstellung der Länge des Implantates gestatten, und zwei spezielle Endplatten, die zur Verankerung des Implantates in den anschliessenden intakten Wirbelkörpern dienen.

Aus der US 4,554,914 KAPP ET AL. ist beispielsweise eine Wirbelkörperprothese bekannt, welche nach dem Entfernen eines defekten Wirbelkörpers zwischen die beiden an diesen angrenzenden gesunden Wirbelkörper eingesetzt wird. Diese bekannte Wirbelkörperprothese umfasst ein Paar längenverstellbarer Stützelemente und Befestigungsmittel. Diese längenverstellbaren Stützelemente werden in den durch die Entfernung eines defekten Wirbelkörpers entstehenden Hohlraum zwischen den benachbarten gesunden Wirbelkörpern eingefügt. Dazu wird nach dem Herstellen des Hohlraumes eine Spreizvorrichtung zwischen die einander gegenüberliegenden Wirbelkörperflächen der benachbarten Wirbelkörper eingesetzt, mittels welcher diese Wirbelkörper distrahiert werden und die normale Höhe des zu ersetzenden Wirbelkörpers wieder hergestellt wird. Die Stützelemente stützen die angrenzenden Wirbelkörper axial und justieren den axialen Zwischenraum zwischen den benachbarten Wirbelkörpern. Zusätzlich wird um die längenverstellbaren Stützelemente ein fliessfähiges und aushärtendes Material eingefügt, so dass die Stützelemente darin eingebettet werden. Die Stützelemente bestehen aus einer Hülse mit einer Bohrung mit Innengewinde und darin einschraubbaren Schrauben. Zudem umfassen die Hülsen am äusseren Ende konische Spitzen während die Schrauben am ihrem äusseren Ende keilförmig gestaltet sind. Durch Expansion der Stützelemente im Hohlraum zwischen den Wirbelkörpern wird erreicht, dass die äusseren Ende von Hülsen und Schrauben in die benachbarten Wirbelkörper eindringen. Die konischen Spitzen gestatten, dass die Hülse drehbar ist, während die keilförmigen äusseren Enden der Schrauben eine Drehung der Schrauben verhindern, so dass die Einstellung der Länge des Stützelementes durch Drehen der Hülse erfolgen kann. Die Befestigungsmittel bestehen aus je zwei länglichen Platten mit Vertiefungen und Schraubenlöchern. Diese Platten werden beidseits der Wirbelsäule an die dorsalen Fortsätze der Wirbelkörper angelegt und verschraubt. Die Schraubenlöcher in den Platten sind so angeordnet, dass die Schrauben die dorsalen Fortsätze der Wirbelkörper durchdringen und somit die Wirbelsäule im Bereich der Wirbelprothese fixieren.

Der Nachteil dieser bekannten Wirbelprothese besteht darin, dass die Stützelemente lediglich bei exakt axialer Belastung eine genügende Stabilität bieten. Ferner ist das Einbringen zweier solcher Stützelemente in den Hohlraum zwischen den angrenzenden gesunden Wirbelkörpern und deren Längenausrichtung mittels der Gewinde zeitraubend.

Eine weitere Vorrichtung für den prothetischen Wirbelkörperersatz ist aus der DE 30 23 942 KELLER bekannt. Diese bekannte Vorrichtung umfasst zwei Teile, welche je ein Stützende zur Anlage an den angrenzenden Wirbelkörper haben und koaxial teleskopierbar sind. Die teleskopierbaren Abschnitte der beiden Teile umfassen eine Stange am ersten Teil und eine komplementäre Bohrung am anderen Teil. Die Stange und die Bohrung weisen einen ovalen Querschnitt auf und bilden eine Passung, welche ein axiales Verschieben der Stange in der Bohrung ermöglicht, wenn die längeren Achsen der ovalen Querschnitt in Deckung sind. Werden die beiden Teile um die Längsachse um 90° gegeneinander verdreht, so dass die längeren Achsen senkrecht zueinander stehen, greifen die an der Stange angebrachten Gewindegänge in diejenigen in der Bohrung ein, wodurch die beiden Teile axial arretierbar sind. Nachteilig an dieser bekannten Vorrichtung ist, dass beide Teile mit den als Stützenden dienenden und an den angrenzenden Wirbelkörpern anliegenden Tellern gegeneinander verdreht werden müssen, so dass die Oberflächen der Wirbelkörper beeinträchtigt werden können.

Der Erfindung liegt die Aufgabe zugrunde, eine stabile, längeneinstellbare und intraoperativ einfach handbare Wirbelprothese zu schaffen, welche die biomechanischen und physiologischen Eigenschaften der Wirbelsäule trotz des entfernten Wirbelkörpers aufrecht erhält.

Die Erfindung löst die gestellte Aufgabe mit einer Vorrichtung, welche die Merkmale des Anspruchs 1 aufweist.

Die erfindungsgemässen Vorrichtung umfasst zwei Hohlzylinder, welche entlang einer Zentralachse ineinander verschiebbar und teleskopierbar sind, und einen hohlzylindrischen, konzentrisch zur Zentralachse der beiden Hohlzylinder angeordneten Fixierring, welcher in einer innerhalb der inneren Mantelfläche des äusseren Hohlzylinders angebrachten Nute um die Zentralachse drehbar gelagert ist. An der äusseren Mantelfläche des inneren Hohlzylinders sind sektoriell Erhebungen angebracht, welche die Form eines Gewindes ohne Steigung aufweisen. Zu diesen Erhebungen korrespondierend sind an der Oberfläche der Bohrung im Fixierring ebenfalls sektoriell Vertiefungen angebracht, so dass beim Eingriff der Erhebungen in die Vertiefungen eine axiale Blockierung der beiden Hohlzylinder gegeneinander hergestellt wird. Die drei Sektoren mit Vertiefungen am Fixierring und diejenigen drei Sektoren mit Erhebungen am inneren Hohlzylinder weisen jeweils einen Sektorwinkel von 60° auf. Alternierend zwischen diesen mit Erhebungen beziehungsweise Vertiefungen versehenen Sektoren liegen Sektoren mit einem Sektorwinkel von ebenfalls 60°, worin am inneren Hohlzylinder keine Erhebungen und am Fixierring keine Vertiefungen angebracht sind und deren Durchmesser so gestaltet sind, dass eine axiale Verschiebung des inneren und des äusseren Hohlzylinders gegeneinander möglich ist. Damit ist gewährleistet, dass in einer ersten Drehwinkelstellung (Position A) des Fixierringes Erhebungen und Vertiefungen miteinander in Eingriff stehen und somit die beiden Hohlkörper in Richtung der Zentralachse relativ zueinander blockiert sind während in einer zweiten Drehwinkelstellung (Position B) des Fixierringes die Erhebungen und Vertiefungen nicht miteinander im Eingriff stehen, so dass die beiden Hohlkörper in Richtung der Zentralachse relativ zueinander frei beweglich sind.

An den freien Enden des inneren und des äusseren Hohlzylinders sind Endplatten angebracht, welche einen grösseren Durchmesser als der innere beziehungsweise der äussere Hohlzylinder aufweisen. Diese Endplatten sind an den zur Anlage an die benachbarten Wirbelknochen bestimmten freien Flächen mit einer dreidimensionale Strukturierung versehen, welche aus konischen oder pyramidenförmigen Spitzen besteht. Bei der Implantation der Vorrichtung wird diese gespreizt, wobei sich diese Spitzen in die Oberflächen der benachbarten Wirbelkörper eingraben und somit das Implantat gegen Verrutschen innerhalb des ausgeräumten Wirbelraumes sichern.

Beide Hohlzylinder sind entlang der Zentralachse durchgehend durchbohrt. Der dadurch erreichte Hohlraum kann mit Knochenspänen aufgefüllt werden, wodurch das Zusammenwachsen der beiden benachbarten Wirbelkörper im Hohlraum der Vorrichtung gefördert wird. Zur Förderung des Anwachsens der benachbarten Wirbelkörper am Implantat könnend die an diese Wirbelkörper angrenzenden Endplatten der Vorrichtung perforiert sein.

Zur Sicherung gegen Verdrehen der beiden Hohlzylinder ist an der inneren Mantelfläche des äusseren Hohlzylinders eine entlang der Zentralachse verlaufende Nut und am inneren Hohlzylinder eine in diese Nut eingreifende Nase angebracht. Damit wird verhindert, dass beim Drehen des Fixierringes von einer Position zur Anderen die Hohlzylinder gegeneinander verdreht werden.

An der äusseren Mantelfläche des Fixierringes ist ein Ansatz angebracht, welcher in einer entsprechenden Aussparung am äusseren Hohlzylinder so bewegbar ist, dass eine Drehbewegung des Fixierringes zwischen den Positionen A) und B) möglich ist und die Nase des Fixierringes in der Position A) an der einen Seitenwand der Aussparung und in der Position B) an der anderen Seitenwand der Aussparung anschlägt. Diese so erreichten seitlichen Anschläge für den Ansatz am Fixierring gestatten ein einfaches Auffinden des ersten Drehwinkels des Fixierringes, bei dem die beiden Hohlzylinder axial fest sind, und des zweiten Drehwinkels des Fixierringes, bei dem die beiden Hohlzylinder axial verschiebbar sind. Zur Sicherung des Fixierringes in einer gewählten Position ist am Ansatz eine in axialer Richtung vorstehende v-förmige Erhebung angebracht, welche in entsprechende Rillen in der Aussparung einrastet, so dass der Fixierring in den Positionen A) und B) lösbar fixierbar ist.

Die durch die Erfindung erreichten Vorteile sind im wesentlichen darin zu sehen, dass dank der erfindungsgemässen Vorrichtung bei axialer Belastung eine hohe Stabilität erreicht wird und die benachbarten Wirbelkörper auf einem ausreichend grossen Querschnitt zusammenwachsen können. Beim Einbringen der Vorrichtung in den ausgeräumten Wirbelraum wird die Vorrichtung mittels einer Spreizzange solange verlängert, bis die Endplatten an den Hohlzylindern an die angrenzenden gesunden Wirbelkörper anstossen und die Spitzen genügend weit in diese Wirbelkörper eindringen. Dazu wird vorangehend der Fixierring in seine zweite Drehwinkelstellung (Position B) gebracht. Nach Erreichen der erforderlichen Länge des Implantates wird der Fixierring mittels eines Stabes, welcher in eine speziell dazu vorgesehen Bohrung im Fixierring einbringbar ist, in seine erste Drehwinkelstellung (Position A) gebracht und somit die Vorrichtung auf der gewünschten Länge blockiert. Diese Längenblockierung des Implantates gewährleistet eine einfache Handhabung bei der Implantation der Vorrichtung.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch die bevorzugte Ausführungsform der erfindungsgemässen Vorrichtung in ihrer minimalen Höhe mit nicht im Eingriff stehenden Kupplungselementen;
Fig. 2 einen Querschnitt durch die in Fig. 1 dargestellte Ausführungsform der erfindungsgemässen Vorrichtung;
Fig. 3 einen Längsschnitt durch die bevorzugte Ausführungsform der erfindungsgemässen Vorrichtung mit vergrösserter Höhe und im Eingriff stehenden Kupplungselementen; und
Fig. 4 einen Querschnitt durch die in Fig. 3 dargestellte Ausführungsform der erfindungsgemässen Vorrichtung.

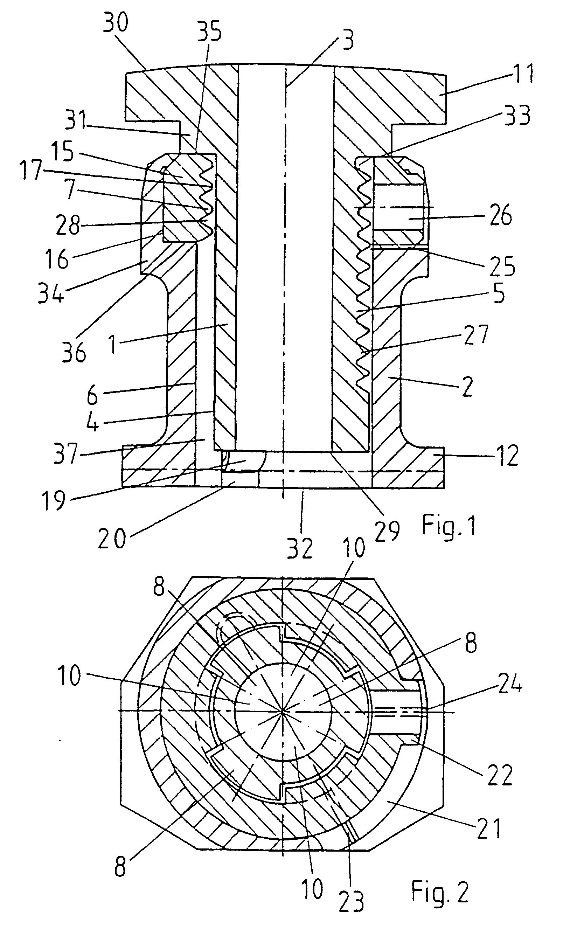

Fig. 1 zeigt die bevorzugte Ausführungsform der erfindungsgemässen Vorrichtung in einem Längsschnitt. Die Vorrichtung umfasst einen inneren Hohlkörper 1, einen äusseren Hohlkörper 2 und einen Fixierring 15. Der innere 1 und der äussere Hohlkörper 2 sind konzentrisch zu einer Zentralachse 3 so angeordnet, dass der innere Hohlkörper 1 im ebenfalls konzentrisch verlaufenden Hohlraum 37 des äusseren Hohlkörpers 2 entlang der Zentralachse 3 verschiebbar ist. Dadurch entsteht eine teleskopierbare Anordnung der beiden Hohlkörper 1;2. In der in Fig. 1 gezeigten Anordnung der Vorrichtung ist der innere Hohlkörper 1 so weit wie möglich in den Hohlraum des äusseren Hohlkörpers 2 eingefahren, wodurch die so dargestellte Vorrichtung ihre geringste mögliche Höhe einnimmt. Der innere Hohlkörper 1 besteht an seinem oberen Ende 30 aus einer polygonförmigen Endplatte 11, einer daran anschliessenden kreiszylindrischen Andrehung 31 und einem zylindrischen Teil mit der äusseren Mantelfläche 4, wobei diese äussere Mantelfläche 4 mit Erhebungen 27 versehen ist, welche als erste Kupplungselemente 5 dienen. Diese Erhebungen 27 sind auf der äusseren Mantelfläche 4 in Sektoren 8 mit einem Winkel von 60° angeordnet, wobei zwischen diesen mit Erhebungen 27 versehenen Sektoren 8 alternierend Sektoren 10 ohne Erhebungen 27 liegen. Die Sektoren 10 schliessen ebenfalls einen Winkel von 60° ein. Die Erhebungen 27 weisen die Form und das Profil eines Gewindes ohne Steigung auf. Der äussere Hohlkörper 2 besteht aus einer weiteren polygonförmigen Endplatte 12 an seinem unteren Ende 32, einem daran anschliessenden kreiszylindrischen Mittelteil und einem ebenfalls kreiszylindrischen, einen grösseren Durchmesser als das Mittelteil aufweisenden Oberteil gegen das obere Ende 33. Der konzentrisch zur Zentralachse 3 verlaufende kreiszylindrische Hohlraum 37 des äusseren Hohlkörpers 2 ist in seinem Durchmesser so bemessen, dass der innere Hohlkörper 1 mit seinem mit den ersten Kupplungselementen 5 versehenen Teil in Richtung der Zentralachse 3 innerhalb dieses Hohlraumes 37 verschiebbar ist. Zudem ist in der Mantelfläche 6 des Hohlraumes 37 des äusseren Hohlkörpers 2 parallel zur Zentralachse 3 eine Nut 20 angebracht, deren Abmessungen so gestaltet sind, dass eine am unteren Ende 29 des inneren Hohlkörpers 1 angebrachte Nase 19 in diese Nut 20 eingreifen kann, womit sich eine Verdrehung der beiden Hohlkörper 1;2 relativ zueinander verhindern lässt. Die axiale Länge der Nut 20 ist so bemessen, dass der innere Hohlkörper 1 auf seiner mit den ersten Kupplungselementen 5 versehenen Länge teleskopierbar ist. Zur Fixierung der axialen Position des inneren Hohlkörpers 1 gegenüber dem äusseren Hohlzylinder 2 ist in eine an der inneren Mantelfläche 6 des äusseren Hohlzylinders 2 konzentrisch zur Zentralachse 3 angebrachten umlaufenden Nut 16 ein Fixierring 15 mit zweiten Kupplungselementen 7 um die Zentralachse 3 drehbar eingefügt. Der Fixierring 15 hat die Form eines zylindrischen Ringes, dessen innere Mantelfläche 17 mit Vertiefungen 28 versehen ist, welche als zweite Kupplungselemente 7 dienen. Diese Vertiefungen 28 sind auf der inneren Mantelfläche 17 in Sektoren 10 mit einem Winkel von 60° angeordnet, wobei zwischen diesen mit Vertiefungen 28 versehenen Sektoren 10 alternierend Sektoren 9 ohne Vertiefungen 28 liegen. Die Sektoren 9 schliessen ebenfalls einen Winkel von 60° ein. Die Vertiefungen 28 weisen die Form und das Profil eines zu den Erhebungen 27 an der äusseren Mantelfläche 4 des inneren Hohlkörpers 1 korrespondierenden Gewindes ohne Steigung auf. In Fig. 1 und 2 sind die am inneren Hohlkörper 1 angebrachten ersten Kupplungselemente 5 und die am Fixierring 15 angebrachten zweiten Kupplungselemente 7 in der Position B) dargestellt. Das heisst, die Kupplungselemente 5;7 stehen miteinander nicht im Eingriff und die beiden Hohlkörper 1;2 sind in Richtung der Zentralachse 3 relativ zueinander frei beweglich. Zum einfacheren Einstellen der Positionen A) und B) ist der Fixierring 15 mit einem Ansatz 22 versehen, welche in eine am äusseren Hohlzylinder 2 angebrachte Aussparung 21 eingreift, wobei diese Aussparung 21 so bemessen ist, das sie eine Drehbewegung des Fixierringes 15 zwischen den Positionen A) und B) zulässt. Zudem ist am Ansatz 22 eine in axialer Richtung vorstehende v-förmige Erhebung 25 angebracht, welcher in entsprechende Rillen 23;24 in der Aussparung 21 einrastet, so dass der Fixierring 15 in den Positionen A) und B) lösbar fixierbar ist. Ebenfalls am Ansatz 22 angebracht ist eine radiale Bohrung 26. Zur Drehung des Fixierringes 15 von der einen Position A);B) in die jeweils andere Position A);B) kann ein gewöhnlicher Dorn (nicht gezeichnet) in die Bohrung 26 eingeführt werden und als Hebelarm benützt werden.

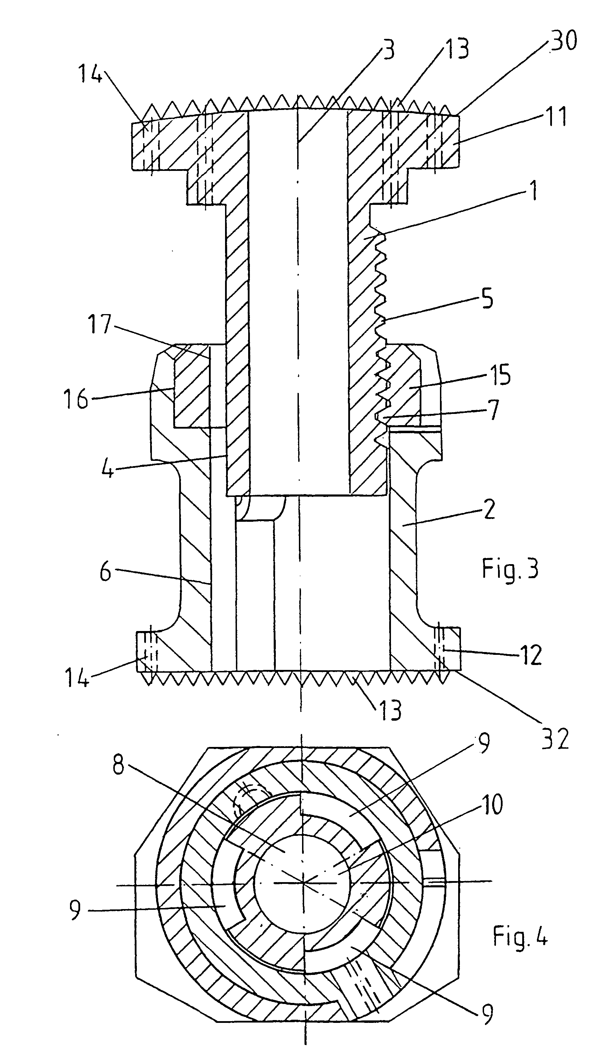

In Fig. 3 ist die erfindungsgemässe Vorrichtung in einer verlängerten und blockierten Position dargestellt. wie in Fig. 4 gezeigt, nimmt dazu der Fixierring die Position A) ein, das heisst, die Kupplungselemente 5;7 sind im Eingriff. In dieser Position A) bilden die Vertiefungen 28 mit den Erhebungen 27 eine formschlüssige Verbindung, welche eine Verschiebung der beiden Hohlkörper 1;2 gegeneinander in Richtung der Zentralachse 3 verhindert. Die in Fig. 3 dargestellte Ausführungsform der erfindungsgemässen Vorrichtung unterscheidet sich sonst von der in Fig. 1 dargestellten Ausführungsform nur dadurch, dass an den freien Endflächen am unteren Ende 32 der äusseren Hohlzylinders 2 und am oberen Ende 30 des inneren Hohlzylinders 1 konische Spitzen 13 angebracht sind, welche in die jeweils angrenzenden gesunden Wirbelkörper eindringen können. Ebenfalls sind beide Endplatten 11;12 perforiert, wodurch ein Anwachsen der angrenzenden Wirbelkörper an die implantierte Vorrichtung gefördert wird.

## Patentansprüche

1. Vorrichtung zum Ersatz von Wirbelkörpern im menschlichen Körper, die einen inneren (1) und einen äusseren (2) longitudinalen Hohlkörper umfasst, welche entlang einer Zentralachse (3) ineinander koaxial gleitbar und in Richtung dieser Zentralachse (3) relativ zueinander verschiebbar sind und der innere Hohlkörper (1) an seiner äusseren Mantelfläche (4) mit ersten Kupplungselementen (5) versehen ist, während im Hohlraum (37) des äusseren Hohlkörpers (2) zweite Kupplungselemente (7) angeordnet sind, welche mit den ersten Kupplungselementen (5) in Eingriff bringbar sind, wobei
die ersten und zweiten Kupplungselemente (5;7) derart zueinander angeordnet sind, dass sie durch relative Verdrehung zueinander wahlweise in mindestens zwei verschiedene Positionen bringbar sind,
einer ersten Position (A), in welcher die ersten und zweiten Kupplungselemente (5;7) miteinander im Eingriff stehen, so dass die beiden Hohlkörper (1;2) in Richtung der Zentralachse (3) relativ zueinander blockiert sind; und
einer zweiten Position (B) in welcher die ersten und zweiten Kupplungselemente (5;7) nicht miteinander im Eingriff stehen, so dass die beiden Hohlkörper (1;2) in Richtung der Zentralachse (3) relativ zueinander frei beweglich sind,
**dadurch gekennzeichnet, dass**
der äussere Hohlkörper (2) zusätzlich einen hohlzylindrischen, konzentrisch zur Zentralachse (3) angeordneten Fixierring (15) umfasst, welcher in einer innerhalb der inneren Mantelfläche (6) angebrachten Nute (16) drehbar gelagert ist, und die zweiten Kupplungselemente (7) am Fixierring (15) angebracht sind.

2. Vorrichtung nach Anspruch 1, **dadurch gekennzeichnet, dass** die ersten Kupplungselemente (5) aus Erhebungen auf der äusseren Mantelfläche (4) und die zweiten Kupplungselemente (7) aus Vertiefungen in der inneren Mantelfläche (6) des äusseren Hohlkörpers (2) bestehen, welche mit den Erhebungen korrespondieren.

3. Vorrichtung nach Anspruch 2, **dadurch gekennzeichnet, dass** die ersten Kupplungselemente (5) auf einen oder mehrere Sektoren (8) der äusseren Mantelfläche (4) beschränkt sind und die zweiten Kupplungselemente (7) auf einen oder mehrere Sektoren (10) der inneren Mantelfläche (6) beschränkt sind, wobei die Sektoren (8;10) auf den beiden Mantelflächen (4;6) den gleichen Winkel aufweisen.

4. Vorrichtung nach Anspruch 3, **dadurch gekennzeichnet, dass** die Sektoren (8;10), innerhalb welcher die Kupplungselemente (5;7) angebracht sind, mit Sektoren (9;18) ohne Kupplungselemente (5;7) alternierend angeordnet sind.

5. Vorrichtung nach Anspruch 1, **dadurch gekennzeichnet, dass** die zweiten Kupplungselemente (7) an der Manteloberfläche der Bohrung (17) des Fixierringes (15) angebracht sind.

6. Vorrichtung nach Anspruch 5, **dadurch gekennzeichnet, dass** die ersten Kupplungselemente (5) aus Erhebungen auf der äusseren Mantelfläche (4) und die zweiten Kupplungselemente (7) aus Vertiefungen in der Manteloberfläche der Bohrung (17) bestehen, welche mit den Erhebungen korrespondieren.

7. Vorrichtung nach Anspruch 5 oder 6, **dadurch gekennzeichnet, dass** die ersten Kupplungselemente (5) auf einen oder mehrere Sektoren (8) der äusseren Mantelfläche (4) beschränkt sind und die zweiten Kupplungselemente (7) auf einen oder mehrere Sektoren (10) der Bohrung (17) beschränkt sind, wobei die Sektoren (8) auf der äusseren Mantelfläche (4) und die Sektoren (10) auf der Manteloberfläche der Bohrung (17) den gleichen Winkel aufweisen.

8. Vorrichtung nach Anspruch 7, **dadurch gekennzeichnet, dass** die Sektoren (8;10), innerhalb-welcher die Kupplungselemente (5;7) angebracht sind, mit Sektoren (9;18) ohne Kupplungselemente (5;7) alternierend angeordnet sind.

9. Vorrichtung nach einem der Ansprüche 1 bis 8, **dadurch gekennzeichnet, dass** beide Hohlkörper (1;2) hohlzylindrisch sind.

10. Vorrichtung nach einem der Ansprüche 1 bis 9, **dadurch gekennzeichnet, dass** die beiden Hohlkörper (1;2) einen nicht kreisförmigen Querschnitt haben.

11. Vorrichtung nach einem der Ansprüche 1 bis 10, **dadurch gekennzeichnet, dass** am freien Ende des inneren Hohlkörpers (1) eine Endplatte (11) angebracht ist.

12. Vorrichtung nach einem der Ansprüche 1 bis 11, **dadurch gekennzeichnet, dass** am freien Ende des äusseren Hohlkörpers (2) eine Endplatte (12) angebracht ist.

13. Vorrichtung nach einem der Ansprüche 11 oder 12, **dadurch gekennzeichnet, dass** die zur Anlage am Wirbelknochen bestimmte freie Fläche der Endplatte (11;12) eine dreidimensionale Strukturierung (13) aufweist.

14. Vorrichtung nach einem der Ansprüche 11 oder 12, **dadurch gekennzeichnet, dass** die zur Anlage am wirbelknochen bestimmte freie Fläche der Endplatte (11;12) Perforierungen (14) aufweist.

15. Vorrichtung nach einem der Ansprüche 5 bis 14, **dadurch gekennzeichnet, dass** der äussere Hohlzylinder (2) an seiner inneren Mantelfläche (6) mit einer entlang der Zentralachse (3) verlaufenden Nut (20) versehen ist und der innere Hohlzylinder (1) eine darin eingreifende Nase (19) aufweist, so dass sich die beiden Hohlzylinder (1;2) nicht gegeneinander verdrehen können.

16. Vorrichtung nach einem der Ansprüche 5 bis 15, **dadurch gekennzeichnet, dass** der Fixierring (15) mit einem Ansatz (22) versehen ist, welcher in eine am äusseren Hohlzylinder (2) angebrachte Aussparung (21) eingreift, wobei diese Aussparung (21) so bemessen ist, das sie eine Drehbewegung des Fixierringes (15) zwischen den Positionen A) und B) zulässt.

17. Vorrichtung nach Anspruch 16, **dadurch gekennzeichnet, dass** am Ansatz (22) eine in axialer Richtung vorstehende v-förmige Erhebung (25) angebracht ist, welche in entsprechende Rillen (23;24) einrastet, so dass der Fixierring (15) in den Positionen A) und B) lösbar fixierbar ist.

## Claims

1. A device replacing vertebras in the human body and comprising an inner longitudinal hollow body (1) and an outer longitudinal hollow body (2), said bodies being able to slide coaxially in each other along a central axis (3) and being displaceable relative to each other in the direction of the central axis (3), the inner hollow body (1) being fitted at its outside surface (4) with first coupling elements (5), second coupling elements (7) being present in the cavity (37) of the outer hollow body (2) and being lockable to the first coupling elements (5), wherein
the first and second coupling elements (5, 7) are so mutually configured that upon their relative rotation they may be moved selectively in at least two different positions,
a first position (A) wherein the first and second coupling elements (5, 7) are mutually engaged whereby the two hollow bodies (1, 2) are mutually locked in the direction of the central axis (3),
a second position (B) wherein the first and second coupling elements (5, 7) are mutually disengaged whereby the two hollow bodies (1, 2) are mutually freely displaceable in the direction of the central axis (3);
**characterized in that**
the outer hollow body (2) additionally comprises a hollow-cylindrical affixation ring (15) mounted concentrically with the central axis (3) and rotatably supported in a groove (16) in the inside surface (6), and **in that** the second coupling elements (7) are present at the affixation ring (15).

2. Device as claimed in claim 1, **characterized in that** the first coupling elements (5) consist of elevations on the outside surface (4) and the second coupling elements (7) consist of recesses in the inside surface (6) of the outer hollow body (2) and match the elevations.

3. Device as claimed in claim 2, **characterized in that** the first coupling elements (5) are restricted to one or more sectors (8) of the outside surface (4) and **in that** the second coupling elements (7) are restricted to one or more sectors (10) of the inside surface (6), the sectors (8 , 10) on the two surfaces (4, 6) subtending the same angle.

4. Device as claimed in claim 3, **characterized in that** the sectors (8, 10) encompassing the coupling elements (5, 7) are configured in alternating manner with sectors (9, 18) lacking any coupling elements.

5. Device as claimed in claim 1, **characterized in that** the second coupling elements (7) are present at the surface of the borehole (17) of the affixation ring (15).

6. Device as claimed in claim 5, **characterized in that** the first coupling elements (5) consist of elevations on the outside surface (4) and **in that** the second coupling elements (7) consist of recesses in the surface of the borehole (17) and match the elevations.

7. Device as claimed in either of claims 5 and 6, **characterized in that** the first coupling elements (5) are restricted to one or more sectors (8) of the outside surface (4) and **in that** the second coupling elements (7) are restricted to one or more sectors (10) of the borehole (17), the sectors (8) on the outside surface (4) and the sectors (10) on the surface of the borehole (17) subtending the same angle.

8. Device as claimed in claim 7, **characterized in that** the sectors (8, 10) containing the coupling elements (5, 7) are configured in alternating manner with sectors (9, 18) lacking coupling elements.

9. Device as claimed in one of claims 1 through 8, **characterized in that** the two hollow bodies (1, 2) are hollow-cylindrical.

10. Device as claimed in one of claims 1 through 9, **characterized in that** the two hollow bodies (1, 2) are of non-circular cross-section.

11. Device as claimed in one of claims 1 through 10, **characterized in that** an end plate (11) is configured at the free end of the inner hollow body (1).

12. Device as claimed in one of claims 1 through 11, **characterized in that** an end plate (12) is configured at the free end of the outer hollow body (2).

13. Device as claimed in either of claims 11 and 12, **characterized in that** the free surface of the end plate (11, 12) coming to rest against the vertebra comprises a three-dimensional structure (13).

14. Device as claimed in either of claims 11 and 12, **characterized in that** the free surface of the end plate (11, 12) coming to rest against the vertebra is fitted with perforations (14).

15. Device as claimed in one of claims 5 through 14, **characterized in that** the outer hollow cylinder (2) comprises at its inside surface (6) a groove (20) running along the central axis (3) and **in that** the inner hollow cylinder (1) comprises a beak (19) engaging said groove (20), whereby the two hollow cylinders (1, 2) are prevented from relative rotation.

16. Device as claimed in one of claims 5 through 15, **characterized in that** the affixation ring (15) comprises an offset (22) engaging a clearance (21) at the outer hollow cylinder (2) and so dimensioned as to allow rotating the affixation ring (15) between the positions (A) and (B).

17. Device as claimed in claim 16, **characterized in that** an axially projecting V-shaped elevation (25) is present at the offset (22) and detents into matching flutings (23, 24) whereby the affixation ring (15) is detachably lockable into the positions (A) and (B).

## Revendications

1. Dispositif pour remplacer les corps vertébraux dans le corps humain, qui comprend un corps creux longitudinal interne (1) et un externe (2) pouvant glisser coaxialement l'un dans l'autre le long d'un axe central (3) et mobiles l'un par rapport à l'autre selon la direction de cet axe central (3), le corps creux interne (1) étant muni de premiers éléments d'accouplement (5) sur sa surface de gaine externe (4) tandis que les deuxièmes éléments d'accouplement (7) pouvant être mis en prise avec les premiers éléments d'accouplement (5) sont placés dans la cavité (37) du corps creux externe (2),
les premiers et deuxièmes éléments d'accouplement (5;7) étant disposés les uns par rapport aux autres de telle sorte qu'une rotation de ces éléments les uns par rapport aux autres permet de les placer dans au moins deux positions différentes au choix,
une première position (A) dans laquelle les premiers et deuxièmes éléments d'accouplement (5;7) sont en prise entre eux, de sorte que les deux corps creux (1;2) sont bloqués l'un par rapport à l'autre selon la direction de l'axe central (3); et
une deuxième position (B) dans laquelle les premiers et deuxièmes éléments d'accouplement (5;7) ne sont pas en prise entre eux, de sorte que les deux corps creux (1;2) sont mobiles librement l'un par rapport à l'autre selon la direction de l'axe central (3),
**caractérisé en ce que**
le corps creux externe (2) comprend en plus un anneau de fixation (15) cylindrique creux concentrique par rapport à l'axe central (3) et tournant sur paliers dans une rainure (16) placée dans la surface de gaine interne (6), et **en ce que** les deuxièmes éléments d'accouplement (7) sont placés sur l'anneau de fixation (15).

2. Dispositif selon la revendication 1, **caractérisé en ce que** les premiers éléments d'accouplement (5) sont constitués de saillies sur la surface de gaine externe (4) et **en ce que** les deuxièmes éléments d'accouplement (7) sont constitués d'encoches dans la surface de gaine interne (6) du corps creux externe (2), qui correspondent aux saillies.

3. Dispositif selon la revendication 2, **caractérisé en ce que** les premiers éléments d'accouplement (5) sont limités à un ou plusieurs secteurs (8) de la surface de gaine externe (4) et **en ce que** les deuxièmes éléments d'accouplement (7) sont limités à un ou plusieurs secteurs (10) de la surface de gaine interne (6), les secteurs (8;10) sur les deux surfaces de gaine (4;6) présentant le même angle.

4. Dispositif selon la revendication 3, **caractérisé en ce que** les secteurs (8;10) dans lesquels sont disposés les éléments d'accouplement (5;7) alternent avec des secteurs (9;18) sans éléments d'accouplement (5;7).

5. Dispositif selon la revendication 1, **caractérisé en ce que** les deuxièmes éléments d'accouplement (7) sont placés sur la surface de gaine de l'alésage (17) de l'anneau de fixation (15).

6. Dispositif selon la revendication 5, **caractérisé en ce que** les premiers éléments d'accouplement (5) sont constitués de saillies sur la surface de gaine externe (4) et **en ce que** les deuxièmes éléments d'accouplement (7) sont constitués d'encoches dans la surface de gaine de l'alésage (17), qui correspondent aux saillies.

7. Dispositif selon la revendication 5 ou 6, **caractérisé en ce que** les premiers éléments d'accouplement (5) sont limités à un ou plusieurs secteurs (8) de la surface de gaine externe (4) et **en ce que** les deuxièmes éléments d'accouplement (7) sont limités à un ou plusieurs secteurs (10) de l'alésage (17), les secteurs (8) sur la surface de gaine externe (4) et les secteurs (10) sur la surface de gaine de l'alésage (17) présentant le même angle.

8. Dispositif selon la revendication 7, **caractérisé en ce que** les secteurs (8;10) dans lesquels sont disposés les éléments d'accouplement (5;7) alternent avec des secteurs (9;18) sans éléments d'accouplement (5;7).

9. Dispositif selon une des revendications 1 à 8, **caractérisé en ce que** les deux corps creux (1;2) sont cylindriques et creux.

10. Dispositif selon une des revendications 1 à 9, **caractérisé en ce que** les deux corps creux (1;2) possèdent une section non circulaire.

11. Dispositif selon une des revendications 1 à 10, **caractérisé en ce que** l'extrémité libre du corps creux interne (1) porte une plaque terminale (11).

12. Dispositif selon une des revendications 1 à 11, **caractérisé en ce que** l'extrémité libre du corps creux externe (2) porte une plaque terminale (12).

13. Dispositif selon une des revendications 11 ou 12, **caractérisé en ce que** la surface libre de la plaque terminale (11;12) destinée à être appliquée sur la vertèbre présente une structure en trois dimensions (13).

14. Dispositif selon une des revendications 11 ou 12, **caractérisé en ce que** la surface libre de la plaque terminale (11;12) destinée à être appliquée sur la vertèbre présente des perforations (14).

15. Dispositif selon une des revendications 5 à 14, **caractérisé en ce que** la surface de gaine interne (6) du cylindre creux externe (2) est munie d'une rainure (20) courant le long de l'axe central (3) et **en ce que** le cylindre creux interne (1) présente une came (19) qui s'enclenche dans cette rainure, de sorte que les deux cylindres creux (1 ;2) ne peuvent pas tourner l'un par rapport à l'autre.

16. Dispositif selon une des revendications 5 à 15, **caractérisé en ce que** l'anneau de fixation (15) est muni d'un épaulement (22) qui s'enclenche dans un évidement (21) placé sur le cylindre creux externe (2), les dimensions de cet évidement (21) permettant un mouvement rotatif de l'anneau de fixation (15) entre les positions A) et B).

17. Dispositif selon la revendication 16, **caractérisé en ce que** l'épaulement (22) est muni d'une saillie en V (25) dépassant dans le sens axial, qui s'enclenche dans des rainures correspondantes (23;24), de sorte que l'anneau de fixation (15) peut être bloqué de façon amovible en position A) et B).
